(19) Europäisches Patentamt
European Patent Office
Office européen des brevets

(11) Numéro de publication: **0 357 519 A1**

(12) **DEMANDE DE BREVET EUROPEEN**

(21) Numéro de dépôt: **89420271.2**

(22) Date de dépôt: **25.07.89**

(51) Int. Cl.[5]: **A 61 K 9/20**
C 08 K 13/02, C 02 F 1/76

(30) Priorité: **29.07.88 FR 8810535**

(43) Date de publication de la demande:
**07.03.90 Bulletin 90/10**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Cyprien, Guy**
**7, rue d'Anjou**
**F-94240 L'Häy-les-Roses (FR)**

**Fisch, Alain**
**3, allée de Longchamp**
**F-92150 Suresne (FR)**

**Haggiage, Johnny**
**72, rue du Fort St-Irénée**
**F-69005 Lyon (FR)**

**Porte, Huges**
**6, chemin de Crépieux**
**F-69300 Caluire (FR)**

**Prazuck, Thierry**
**53, av. Mathurin-Moreau**
**F-75019 Paris (FR)**

**Torres, Ghislaine**
**104, rue Ney**
**F-69006 Lyon (FR)**

(74) Mandataire: **Seugnet, Jean Louis et al**
**RHONE-POULENC CHIMIE Service Brevets Chimie**
**Centre de Recherches des Carrières B.P. 62**
**F-69192 Saint-Fons Cédex (FR)**

(54) **Composition organopolysiloxane contenant de l'iode, transformable à chaud en élastomère utilisable pour le traitement des eaux.**

(57) La présente invention concerne une composition de silicone comportant :
- une huile diorganopolysiloxane, de viscosité inférieure à 300 000 mPa.s à 25°C,
- une charge minérale,
- un peroxyde organique,
- au moins un composé organique et/ou minéral de l'iode sous forme solide ou liquide à température ambiante, soluble dans l'eau et non toxique.

Les élastomères obtenus après action de la chaleur sur ces compositions sont utilisables pour le traitement des eaux avec libération en continu et contrôlée de l'iode pour le traitement et l'éradication des maladies dues à une carence en iode.

EP 0 357 519 A1

## Description

## COMPOSITION ORGANOPOLYSILOXANE CONTENANT DE L'IODE, TRANSFORMABLE A CHAUD EN ELASTOMERE UTILISABLE POUR LE TRAITEMENT DES EAUX

Le présente invention concerne une composition de silicone à base d'huile(s) diorganopolysiloxane(s) contenant de l'iode, l'élastomère obtenu avec cette composition après qu'elle ait été chauffée, ainsi qu'un procédé de traitement des eaux d'usage domestique et de boissons à l'aide de cet élastomère.

On estime actuellement à plusieurs centaines de millions le nombre de sujets présentant und déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc...).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'alors sont demeurées inefficaces.

Ces actions visent essentiellement d'une part :

- l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajoutée au sel s'en échappe rapidement lorsqu'il n'est pas parfaitement emballé.

et d'autre part :

- l'injection intramusculaire d'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induites par l'injection d'une dose nécessairement supra-physiologique.

On a par ailleurs décrit dans le brevet belge BE-A-889 680 l'introduction d'oligo-éléments, dont l'iode, dans l'eau de boisson des ruminants, sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A 2 347 567, US-A-2 743 208 et US-A-3 408 295.

Il existe également de très nombreux brevets décrivant l'utilisation de systèmes polymères, en particulier de silicone, pour la libération contrôlée d'un principe actif au moyen par exemple d'un système transdermique (brevet américain US-A-4 053 580), ou par absorption buccale notamment pour des ruminants (brevet français FR-A-2 560 768).

Enfin par le brevet américain US-A-4 384 960 il est décrit la mise en pastilles diode $I_2$ dans une bouteille en plastique dans laquelle l'eau pénètre par une membrane poreuse de polymère. L'eau solubilise l'iode. Le rôle de la membrane est seulement d'éviter que l'iode en pastilles ne sorte de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car d'une par $I_2$ est un inhibiteur bien connu des catalyseurs de durcissement des élastomères silicones vulcanisables à température ambiante (voir en particulier la publication de W.D. MORAIN et al. Plastic and Reconstructive Surgery 59, 2, 215-222 (1977), et d'autre part du fait de sa volatilité élevée, $I_2$ se sublime lors de la réticulation à chaud des élastomères silicones.

Toutefois das ce système il n'y a pas de contrôle de la libération de l'iode d'une part, et, d'autre part, l'iodation de l'eau se fait par addition discontinue ou en continu de quelques gouttes d'eau très iodée (à saturation) contenue dans la bouteille, dans un récipient quelconque contenant de l'eau non traitée. Il est clair que la solution proposée par US-A-4 384 960 est imparfaite, par le fait notamment qu'il s'agit d'une technique individuelle qui, comme l'injection intra-musculaire d'iode, nécessite une éducation et une mobilisation massive des populations.

La présente invention a précisément pour but de proposer une composition de silicone contenant de l'iode, qui après chauffage (c'est-à-dire sa transformation en élastomère) est utilisable pour le traitement continu des eaux d'usage domestique, en particulier dans les systèmes d'adduction et de traitement des eaux dans les puits et les forages et qui permette de relarguer (libérer) une quantité contrôlée et adaptée d'iode en vue d'assurer d'une part le traitement collectif des diverses manifestations dues à une carence en iode et d'autre part une prophylaxie de ces diverses manifestations.

Elle a également pour but de proposer une composition de silicone contenant de l'iode qui après voir été chauffée (transformée en élastomère) et immergée de façon appropriée dans les endroits contenant l'eau à traiter, en particulier les puits et forages, relargue (libère) de façon continue, de préférence pendant au moins un an, une quantité appropriée d'iode sous une forme et à une dose thérapeutiquement active et efficace pour soigner les diverses maladies dues à une carence en iode.

Elle a également pour but de proposer une composition de silicone contenant de l'iode qui, immergée (sous forme d'élastomère) de façon appropriée dans les endroits contenant l'eau à traiter n'ait aucune action secondaire indésirable et néfaste sur le plan chimique et biologique sur les eaux à traiter.

Elle a également pour but de proposer une composition de silicone contenant de l'iode présentant (après avoir été transformée en élastomère) une forme adaptée au milieu dans lequel se trouve l'eau à traiter, cette forme étant adaptée en particulier aux puits et/ou forages et présentant un système d'introduction dans les puits et/ou forages permettant son remplacement aisé.

Elle a également pour but de proposer une composition de silicone pâteuse et pompable, au moins avant l'incorporation du composé iodé.

Ces buts et d'autres sont atteints par la présente invention qui concerne en effet une composition de silicone comportant :

A) une huile diorganopolysiloxane, de viscosité inférieures à 300 000 mPa.s à 25° C,

B) une charge minérale

C) un peroxyde organique et

D) au moins un composé organique et/ou minéral de l'iode sous une forme solide ou liquide à température ambiante, soluble dans l'eau et non toxique.

La présente invention concerne également l'élastomère obtenu avec la composition selon la présente invention (après que cette composition ait été chauffée) et le procédé mis en oeuvre avec ledit élastomère notamment pour le traitement des eaux d'usage domestique.

Comme composé minéral de l'iode, à l'exception de l'iode moléculaire $I_2$, on peut utiliser seul ou en mélange :

- les iodures ou iodates de formules générales :

$(M^{a+})\ (I^-)_a$

et $(M^{a+})\ (IO_3^-)_a$

dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NY_4)^+$, dont les radicaux Y identiques ou différents représentent un radical alkyle linéaire ou ramifié en $C_1$-$C_{20}$ ou un atome d'hydrogène, tel que l'ion ammonium $NH^+_4$.

Les cations $M^{a+}$ et $NY_4^+$, sont choisis de telle sorte que l'iodure ou l'iodate correspondant soit un solide ou un liquide à température ambiante, soit soluble dans l'eau et soit non toxique.

comme iodures et iodates on peut en particulier utiliser ceux de formules :

$Na\ I$ , $Na\ IO_3$,

$K\ I$ , $K\ IO_3$,

$Mg\ I_2$ , $MG\ I_2$, $8H_2O$,

$Mg(IO_3)_2$, $4H_2O$,

$NH_4I$

$Fe\ I_2$, $4H_2O$

$Mn\ I_2$

Ces sels peuvent contenir de l'eau d'hydratation ou de l'eau de constitution.

Comme composé de l'iode à la fois organique et minéral on peut par exemple utiliser l'iodobéhénate de calcium de formule :

$(C_{21}H_{42}ICO_2)Ca$

Comme composé organique de l'iode on peut citer la polyvinylpyrrolidone iodée.

Pour des raisons de facilité de mise en oeuvre les composés d'iode solides sont préférés, et parmi ceux-ci NaI et $KIO_3$ sont les plus préférés.

Tous les composés de l'iode tels que définis ci-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 500 mg/l à température ambiante.

On utilise généralement de 5 à 130 parties de préférence de 10 à 90 parties de composé de l'iode (D) pour 100 parties d'huile (A).

En particulier dans les pays en développement, l'eau à usage domestique (boisson, lavage, irrigation, etc...) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'un colonne de 10 à 70 mètres, généralement de 30 à 50 mètres de haut qui est extraite par exemple à l'aide d'une pompe à corps immergé.

Cette eau se renouvelle principalement en fonction de l'utilisation du forage qui dépend de la saison. En effet en saison des pluies le forage est traditionnellement moins utilisé. Par contre en saison sèche, le forage

débite environ 10-12 heures par jour, soit une quantité comprise entre 5 et 10 m3 par jour pendant environ six mois.

En règle générale, un puits peut être asséché deux fois durant la journée au moment de la saison sèche ce qui correspond avec ces données statistiques moyennes, à un maximum d'utilisation de 5 à 10 m3.

De nombreuses études montrent que dans les zones de grande endémie goîtreuse, le taux pré-existant d'équivalent en iode dans l'eau des forages ou des puits est inférieur à 2 microgrammes par litre (2 µg/l). Il est estimé actuellement qu'un apport journalier d'environ 100 µg d'équivalent iode par jour et par personne serait suffisant pour prévenir le développement d'un goître endémique et sans doute environ 150 µg lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 µg de préférence environ 100 µg, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 µg/l d'iode ce qui correspond à 50 µg, d'équivalent iode par litre et par personne, ce qui nécessite que l'élastomère de silicone relargue 720 mg/j d'équivalent d'iode, soit 270 g d'équivalent iode à relarguer pendant une année.

Le système de libération contrôlée de l'iode fait partie des systèmes matriciels dont la diffusion du principe actif est normalement régie par la loi de FICK, c'est-à-dire par une cinétique de diffusion d'ordre 1/2 pour seulement 60 % en poids du principe actif. Au delà de 60 % il y a épuisement de la matrice et les flux de diffusion sont fortement diminués. De façon surprenante et inattendue, on a trouvé que le système matriciel silicone conforme à l'invention libère l'iode suivant une cinétique d'ordre O et de façon continue et cela jusqu'à ce que 80 % en poids et plus du composé d'iode soit libéré.

L'avantage considérable apporté par la matrice silicone est donc qu'il est très facile d'extrapoler la diffusion continue du principe actif après une mesure de la quantité libérée au bout d'au moins un mois puisque l'on sait que la cinétique de diffusion est d'ordre O et qu'au moins 80 % du composé d'iode sera libéré suivant cette cinétique.

De façon à maîtriser la libération du principe actif, il est avantageux de présenter la matrice silicone sous la forme de modules (éléments) élémentaires de formes variées tel que des cubes, des parallélépipèdes rectangles, des cylindres, des sphères dont les paramètres fondamentaux sont les suivants :

- la nature du composé d'iode,
- le diamètre moyen (granulométrie) g des particules du composé d'iode dans le cas préféré où ce dernier est un solide,
- la teneur t de composé d'iode au sein de la matrice,
- le rapport R de la surface au volume du module.

La nature du composé d'iode et sa granulométrie définissent la vitesse de diffusion v à travers la matrice, du principe actif.

Plus g est petit et plus v est lent et inversement.

Plus t est grand et plus le flux de principe actif est élevé et inversement.

Plus R est grand et plus le flux élevé de principe actif est grand et inversement.

L'homme de métier, par des expériences de routine, peut sans difficulté aboutir rapidement au résultat visé en extrapolant le temps d'élution théorique qui correspondra au temps réel de diffusion du principe actif.

Pour NaI et $KIO_3$ qui sont les composés d'iode préférés, g, t et R peuvent être avantageusement choisis dans les zones suivantes :

- g compris entre 1 et 300 µm,
- t compris entre 10 et 100 parties en poids de composé d'iode pour 100 parties de (A),
- R compris entre 0,5 et 50 pour une forme cylindrique.

Il est en outre souhaitable que le composé d'iode soit dispersé de façon homogène au sein de la matrice.

Dans tout ce qui suit ou ce qui précède, sauf indications contraires, les parties et pourcentages sont en poids.

Plus précisément la présente invention concerne une composition de silicone comportant :

- (A) 100 parties d'une huile (ou d'un mélange d'huiles) diorganopolysiloxane ayant une viscosité inférieure à 300 000 mPa.s à 25° C.
- (B) 5 à 90 parties d'une charge minérale, de préférence silicieuse, choisie parmi les silices de pyrogénation et les silices de précipitation,
- (C) 0,1 à 6 parties d'un peroxyde organique,
- (D) 5 à 130 parties, de préférence 10 à 90 parties d'un composé organique et/ou minéral de l'iode, solide à température ambiante, soluble dans l'eau et non toxique.
- (E) 0 à 20 parties, de préférence 1 à 15 parties, d'un agent antistructure.

De façon avantageuse les compositions telles que celle définie ci-avant ont une pénétration s'étalant de 80 à 400 (mesurée selon la norme NF T 60-132) avant l'incorporation du composé organique et/ou minéral de l'iode. De telles compositions sont pâteuses et pompables, au moins avant l'incorporation du composé iodé.

L'huile citée en A (ou le mélange d'huiles) a une viscosité comprise entre 500 et 300 000 m.Pa.s à 25° C, de

préférence comprise entre 800 et 250 000 m.Pa.s à 25° C. Ces huiles sont des polymères linéaires constitués essentiellement d'une succession de motifs de formule $R_2SiO$ et bloqués à chaque extrémité de leur chaîne par des motifs de formule $R_2R'SiO_{0,5}$, dans lesquelles les symboles R, identiques ou différents, représentent des radicaux hydrocarbonés, ayant de 1 à 8 atomes de carbone, substitués ou non par des atomes d'halogènes et/ou des groupes cyano, le symbole R' ayant la signification des symboles R ou représentant un radical hydroxyle, alcoxyle ayant de 1 à 4 atomes de carbone, β-méthoxyéthyle. Il n'est pas exclu cependant que ces huiles renferment de faibles quantités, représentant au plus 1 % en nombre, de motifs de formules $RSiO_{1,5}$ et/ou $SiO_2$, R ayant la signification donnée ci-dessus.

Il doit être entendu que lorsque la composition comprend un mélange d'huiles en (A), une de ces huiles peut avoir une viscosité supérieure à 300 000 m.Pa.s, par exemple comprise entre 300 000 et 500 000 mPa.s à 25° C, mais que le mélange des huiles n'a pas une viscosité résultante supérieure à 300 000 mPa.s à 25°C.

Les radicaux hydrocarbonés, substitués ou non par des atomes d'halogènes ou des groupes cyano, ayant de 1 à 8 atomes de carbone, représentés par les symboles R englobent :

- les radicaux alcoyles et halogénoalcoyles ayant de 1 à 3 atomes de carbone tels que les radicaux méthyles, éthyles, propyles, isopropyles, trifluoro-3,3,3 propyles
- les radicaux alcényles ayant de 2 à 4 atomes de carbone tels que les radicaux vinyles, allyles, butène-2 yles
- les radicaux cycloalcoyles et halogènocycloalcoyles, ayant de 5 à 6 atomes de carbone nucléaire, tels que les radicaux cyclopentyles, cyclohexyles, méthylcyclohexyles, chlorocyclohexyles
- les radicaux aryles et halogénoaryles mononucléaires, ayant de 6 à 8 atomes de carbone tels que les radicaux phényles, tolyles, xylyles, chlorophényles, dichlorophényles, trichlorophényles
- les radicaux cyanoalcoyles dont les restes alcoyles ont de 2 à 3 atomes de carbone tels que les radicaux β-cyanoéthyles, γ-cyanopropyles.

Comme exemples concrets de radicaux alcoxyles, ayant de 1 à 4 atomes de carbone, représentés par les radicaux R', peuvent être cités les radicaux méthoxyles, ethoxyles, n-propoxyles, isopropoxyles, n-butoxyles.

A titre illustratif de motifs constituant essentiellement les huiles diorganopolysiloxaniques A, peuvent être cités respectivement les motifs $R_2SiO$ de formules :
$(CH_3)_2SiO$ , $(CH_3)(CH_2=CH)SiO$ $CH_3(C_6H_5)SiO$ , $(C_6H_5)_2SiO$ , $(C_6H_5(CH_2=CH)SiO$ $CF_3CH_2CH_2(CH_3)SiO$ , $NC\text{-}CH_2CH_2(CH_3)SiO$ , $NC(CH_2)_3CH_3CH_3SiO$ et les motifs $R_2R'SiO_{0,5}$ de formule :
$(CH_3)_3SiO_{0,5}$ , $CH_2=CH(CH_3)SiO_{0,5}$ , $(CH_3)_2C_6H_5SiO_{0,5}$ $CH_3(C_6H_5)(CH_2=CH)SiO_{0,5}$ $HO(CH_3)_2SiO_{0,5}$ , $HO(CH_3)(CH_2=CH)SiO_{0,5}$ $CH_3O(CH_3)_2SiO_{0,5}$ , $CH_3CH_2O(CH_3)_2SiO_{0,5}$ $CH_3OCH_2CH_2O(CH_3)_2SiO_{0,5}$

De préférence sont utilisées des huiles diméthylpolysiloxaniques contenant une faible quantité de radicaux vinyles représentant par exemple de 0,005 à 0,5 % du poids des huiles ; ces radicaux vinyles sont présents sous forme de motifs de formules $CH_3(CH_2=CH)SiO$ et/ou $(CH_3)_2CH_2=CHSiO_{0,5}$ et les huiles sont bloquées par des motifs de formules $(CH_3)_3SiO_{0,5}$ et/ou $(CH_3)_2CH_2=CHSiO_{0,5}$.

Les huiles diorganopolysiloxaniques citées en A sont commercialisées par les fabricants de silicones ; d'autre part elles peuvent être aisément fabriquées en suivant les techniques déjà connues. Ainsi l'une des plus courantes consiste à polymériser des diorganocyclopolysiloxanes à l'aide de quantités catalytiques d'agents alcalins ou acides. Au cours de cette polymérisation sont ajoutés :

1 - (quand R' = R), des diorganopolysiloxanes de faible poids moléculaire de formule $R_3SiO(R_2SiO)_xSiR_3$
x ayant une valeur suffisante pour conduire à une viscosité s'étalant de 0,5 à 100 mPa.s à 25° C.

2 - (quand R' = OH), de l'eau et/où une huile de formule $HOR_2SiO(SiR_2O)_ySiR_2OH$
y ayant une valeur suffisante pour conduire à une viscosité s'étalant de 5 à 200 mPa.s à 25° C.

3 - (quand R' = alcoxyle ou $CH_3OCH_2CH_2O$), l'alcool correspondant R'H et/ou une huile de faible poids moléculaire de formule $R'R_2SiO(R_2SiO)_zSiR_2R'$
z ayant une valeur suffisante pour conduire à une viscosité s'étalant de 0,5 à 120 mPa.s à 25° C.

Les polymères obtenus sont, de préférence, purifiés en éliminant, à une température en général supérieure à 70° C et sous une pression en général inférieure à la pression atmosphérique, les composés de départ non transformés équilibrant la réaction de polymérisation ainsi que les polymères de faible poids moléculaire éventuellement formés lors de cette réaction. Il est recommandé avant de distiller les produits volatils de neutraliser les agents alcalins ou acides utilisés comme catalyseurs de polymérisation.

Les charges minérales citées en B sont utilisées à raison de 5 à 90 parties, de préférence 10 à 80 parties, pour 100 parties de l'huile citée en A.

Ces charges peuvent se présenter sous la forme de produits très finement divisés dont le diamètre particulaire moyen est inférieur à 0,1 micromètre. Parmi ces charges figurent les silices de combustion et les silices de précipitation ; leur surface spécifique BET est généralement supérieure à 40 m2/g.

Ces charges peuvent également se présenter sous la forme de produits plus grossièrement divisés, de diamètre particulaire moyen supérieur à 0,1 micromètre. Comme exemples de telles charges, on peut citer le quartz broyé, les silices de diatomées, l'argile calcinée, de leur surface spécifique est généralement inférieure à 30 m2/g.

Ces charges (B) peuvent avoir été modifiées en surface par traitement avec les divers composés organosiliciques habituellement employés pour cet usage. Ainsi ces composés organosiliciques peuvent être des organochlorosilanes, des diorganocyclopolysiloxanes, des hexaorganodisiloxanes, des hexaorganodisilazanes ou des diorganocyclopolysiloxanes (brevets français 1 126 884, 1 136 885, 1 236 505 ; brevet anglais 1 024 234). Les charges traitées renferment, dans la plupart des cas, de 3 à 30 % de leur poids de composés

organosiliciques.

Les charges (B) peuvent être constituées d'un mélange de plusieurs types de charges de granulométrie différente ; ainsi par exemple, elles peuvent être constituées de 30 à 70 % de silices finement divisées de surface spécifique BET supérieure à 40 m2/g et de 70 à 30 % de silices plus grossièrement divisées de surface spécifique inférieure à 30 m2/g.

Les quantités de silices traitées sont avantageusement présentes à raison d'au moins 5 %, de préférence 7 %, des quantités de silices utilisées.

Les agents antistructures cités en E sont utilisés à raison de 0 à 20 parties, de préférence 1 à 15 parties, pour 100 parties des huiles diorganopolysiloxaniques citées en A. Leur présence empêche les compositions de subir une évolution au cours de temps, laquelle évolution se traduit généralement par une diminution de la valeur des pénétrations.

Ils sont choisis le plus souvent parmi :

- les huiles diorganopolysiloxaniques de faibles viscosités, de l'ordre de 5 à 500 mPa.s à 25°C, bloquées à chaque extrémité de leur chaîne par un radical hydroxyle et/ou un radical alcoxyle ayant de 1 à 3 atomes de carbone. Les radicaux organiques, liés aux atomes de silicium de ces huiles, sont, de préférence, des radicaux méthyles, éthyles, vinyles, phényles, trifluoro-3,3,3 propyles.

A titre d'exemples concrets de ces huiles peuvent être citées les huiles
αω dihydroxydiméthylpolysiloxaniques,
αω dihydroxyméthylphénylpolysiloxaniques,
αω diméthoxydiméthylpolysiloxaniques et
αω diméthoxyméthylphénylpolysiloxaniques, ayant de 3 à 12 % de radicaux hydroxyles ou méthoxyles.

- le diphénylsilanediol et les silanes de formules :

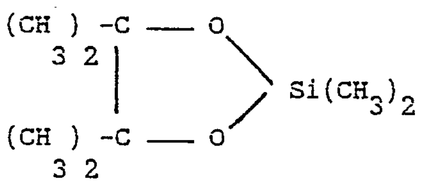 , 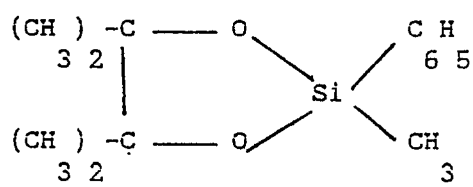

Les peroxydes organiques cités en (C) sont utilisés à raison de 0,1 à 6 parties, de préférence 0,15 à 4 parties, pour 100 parties de l'huile (A). Ce sont des peroxydes organiques habituellement employés pour le durcissement des élastomères silicones vulcanisés à chaud. Ces peroxydes comportent plus spécialement le peroxyde de benzoyle, le peroxyde de dichloro-2,4 benzoyle, le peroxyde de dicumyle, le bis(t-butylperoxy)-2,5 diméthyl-2,5 hexane, le perbenzoate de t-butyle, le peroxyde de di-t-butyle, le peroxyde de cumyle et de t-butyle, le carbonate de t-butylperoxyde et d'isopropyle, le bis(t-butylperoxy)-1,1 triméthyl-3,3,5 cyclohexane, le peroxyde de cumyle et d'α α′ diméthyl p-méthylbenzyle, l'αα′ bis(t-butylperoxy)diisopropylbenzène.

Ces peroxydes se décomposent à des températures et à des vitesses parfois différentes. Ils seront donc choisis en fonction de la technique de durcissement adoptée pour les compositions conformes à l'invention.

D'autre adjuvants peuvent être utilisés aux compositions selon la présente invention dans le dessein d'améliorer leut travaillibilité ou les propriétés mécaniques des élastomères obtenus. Ces adjuvants englobent par exemple les silanes de formules ci-après, et leurs produits d'hydrolyse ou de cohydrolyse partielle :

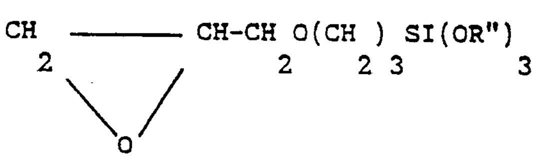

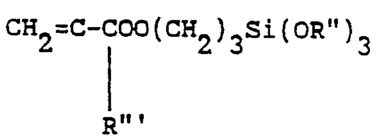

$CH_2=CH\text{-}Si(OR'')_3$

Les symboles R″ représentent les radicaux méthyles, éthyles, n-propyles, β -methoxyéthyles ; le symbole R‴ représente un atome d'hydrogène ou le radical méthyle.

A titre d'exemples concrets de ces silanes peuvent être cités ceux de formules :

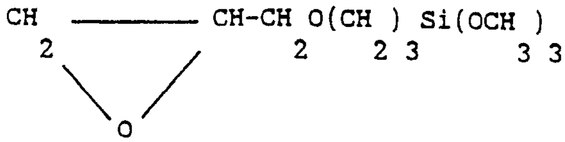

$CH_2=C(CH_3)COO(CH_2)_3Si(OCH_3)_3$
$CH_2=CHSi(OCH_2CH_2OCH_3)_3$

Ces adjuvants sont utilisés à raison de 0 à 5 parties, de préférence 0,05 à 4 parties, pour 100 parties des huiles diorganopolysiloxaniques A.

La préparation des compositions conformes à l'invention s'effectue par le mélange intime des divers constituants A, B, C, D et E. Ce mélange a lieu dans les appareils appropriés utilisés par les fabricants de caoutchouc. Toutefois, étant donné le choix des constituants, il est beaucoup plus facile d'arriver à des mélanges homogènes avec des moyens moins puissants et pendant un temps plus court que pour la préparation de compositions organopolysiloxaniques usuelles renfermant, à la place des huiles A, des gommes diorganopolysiloxaniques.

En particulier les mélangeurs à cylindres ne sont pas utilisables, ce qui est un avantage puisqu'ils consomment du temps et de l'énergie. Par contre peuvent être employés des pétrins, ou des mélangeurs à fourreaux cylindriques équipés de vis, opérant en marche continue ou discontinue ; dans ces mélangeurs à fourreaux cylindriques les vis tournent et peuvent également être soumises à des mouvements de va-et-vient.

L'ordre d'introduction des divers constituants dans les appareils précités peut être quelconque. Néanmoins lorsque les mélanges des constituants sont chauffés à des températures supérieures à 60-80°C, dans le dessin d'accélérer, par exemple, le mouillage des charges B par les huiles A et/ou par l'agent antistructure cité en E (ce qui permet de réduire le temps d'occupation des appareils), il est alors nécessaire d'ajouter le peroxyde organique C en dernier et seulement lorsque la température des mélanges est redescendue à un niveau convenable. Il est en général préférable également d'introduire le composé de l'iode à une température comprise entre 20 et 60°C.

Les compositions selon la présente invention sont aisément manipulables et en particulier peuvent être introduites dans les systèmes d'alimentation des presses à mouler, par simple pompage à l'aide de pompes, par exemple à piston. Elles peuvent être ensuite durcies en pièces de toutes formes et dimensions par les techniques de moulages traditionnelles telles que le moulage par transfert, par compression, par injection, étant entendu que le matériel de moulage employé n'a pas besoin d'être aussi lourd et coûteux que celui employé avec les compositions organopolysiloxaniques caoutchouteuses (ce matériel lourd et coûteux est décrit en particulier dans l'ouvrage de W. LYNCH intitulé "Handbook of silicon rubber fabrication" pages 43 à 48) ; un matériel de moulage léger du type de celui utilisé avec les matières plastiques de basse et moyenne viscosité à l'état fondu, est suffisant. Les moules des presses, pendant l'opération de durcissement des compositions, sont en général portés à une température supérieure à 100°C et inférieure à 250°C.

Les élastomères obtenus avec les compositions selon la présente invention conviennent parfaitement pour l'utilisation souhaitée, c'est-à-dire le traitement des eaux.

Les exemples suivants illustrent l'invention.

EXEMPLE 1 :

1.1 - Préparation de l'élastomère :

On charge dans un pétrin :

- 100 parties d'une huile αω bis(diméthylvinylsiloxy) diméthylpolysiloxane de viscosité 100 000 mPa.s à 25°C, comportant 480 ppm (parties par million) de groupements vinyle.
- 8 parties d'une huile diméthylpolysiloxane de viscosité 400 000 mPa.s à 25°C et contenant 2 % en poids de groupements vinyle dans sa chaîne.
- 4 parties d'une huile αω dihydroxydiméthylpolysiloxane de viscosité 50 m.Pa.s à 25°C.
- 0,3 partie d'un silane de formule :
$CH_2=C(CH_3)COO(CH_2)Si(OCH_3)_3$
- 9 parties de silice de précipitation ayant une surface spécifique de 170 m2/g et commercialisée par la Société DEGUSSA sous la marque FK 320 DS.
- 25 parties de silice de combustion de marque AEROSIL 300, et ayant une surface spécifique de 60 m2/g traitée par de l'octaméthyl cyclotétrasiloxane.
- 10 parties de quartz broyé (référence SIFRACO)
- 0,5 parties de $Ca(OH)_2$

Le mélange soumis à une malaxage efficace, est chauffé progressivement jusqu'à 150°C, puis est malaxé à cette température pendant une heure. Après refroidissement vers 30°C on introduit 30 parties d'iodure de sodium de granulométrie comprise entre 100 et 200 microns, puis après homogénéisation, on ajoute au mélange 0,6 partie de bis (t - butylperoxy)-2,5 diméthyl - 2,5 hexane et on malaxe pendant 5 minutes à cette température de 30°C.

La composition obtenue est ensuite moulée en un boudin (ou jonc) de 9,7 mm de diamètre qui est ensuite durci dans un four à 170°C pendant 15 minutes. On recuit ensuite ce jonc uniforme pendant 4 heures a 200°C.

Le produit résultant est un élastomère de forme cylindrique.

1.2 - Mesure de la cinétique d'élution

L'élastomère contenant le NaI est découpé à la longueur desirée (22mm), conformément au rapport Surface/Volume (5cm-1) que l'on désire atteindre et immergée dans un récipient contenant 250 ml d'eau distillée, thermostatée à 20° C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de l'eau.

Des prélèvements journaliers de 1 ml sont réalisés dans les premiers temps de l'élution, et hebdomadaires au bout de 15 jours d'élution.

La concentration en iodure, libérée par jour, est déterminée par un dosage avec une électrode spécifique à iodure :

A un millilitre de prélèvement du récipient on rajoute deux millilitres d'une solution ($K_2SO_4$- tampon ionique) et un millilitre d'eau distillée. On immerge l'électrode dans cette solution et on lit le potentiel électrochimique de la solution.

Une courbe d'étalonnage préalablement établie avec des solutions d'iodure de $5.10^{-5}$ M/l (M : mole) à $5.10^{-2}$ M/l permet de calculer la concentration (C) en iodure en mg/l de la solution.

Les caractéristiques du cylindre immergé sont :

. Diamètre = 9,7 mm
. Hauteur = 22 mm
. Surface = 9,2 cm2
. Volume = 1,64 $cm^3$
. S/V = 5 $cm^{-1}$
. Masse totale = 2,57 g
. Quantité initiale de I (Qo) = 0,652 g

Dans le tableau suivant (Tab.1) sont rassemblés les résultats de la cinétique d'élution.

Q cumulé correspond à la quantité d'équivalent I (que nous appelons ion actif) éluée à l'instant t.

Sachant que 80 % en moles de l'ion actif incorporé élue selon une cinétique d'ordre zéro en fonction du temps, nous pouvons calculer le temps d'élution théorique (Te) selon :

$$Te = \frac{0,8 \times Qo}{\text{Flux journalier}} \quad \text{(jour)}$$

La courbe Q/Qo = f(t), ainsi que le Te de l'exemple sont reportés sur la figure 1.

Exemple 2 :

La composition selon l'exemple 1 est moulée pour obtenir un jonc de 22,3 mm de diamètre qui est transformée en élastomère selon le mode opératoire décrit dans cet exemple 1.

On immerge dans 500 ml d'eau distillée, thermostatée à 20° C, un cylindre dont les caractéristiques sont les suivantes :

. Diamètre = 22,3 mm
. Hauteur = 57 mm
. Surface = 47,7 cm2
. Volume = 22,25 cm3
. S/V = 2.14 cm-1
. Masse totale = 33,7 g
. Quantité initiale de I (Qo) = 8,55 g

Le tableau 2 rassemble les résultats de la cinétique d'élution. La courbe Q/Qo = f(t), ainsi que le Te de l'exemple sont reportés sur la figure 1 en annexe.

TABLEAU 1

| TEMPS (JOUR) | Q cumulé (gramme) ion acti | 100*Q/QO % |
|---|---|---|
| 1.00 | 0.074 | 11.23 |
| 3.00 | 0.057 | 8.72 |
| 4.00 | 0.070 | 10.72 |
| 8.00 | 0.086 | 13.16 |
| 15.00 | 0.101 | 15.42 |
| 28.00 | 0.125 | 19.04 |
| 43.00 | 0.151 | 23.06 |
| 58.00 | 0.177 | 27.12 |
| 79.00 | 0.209 | 31.97 |

TABLEAU 2

| TEMPS (JOUR) | Q cumulé (gramme) ion acti | 100*Q/QO % |
|---|---|---|
| 1.00 | 0.038 | 0.44 |
| 3.00 | 0.141 | 1.64 |
| 4.00 | 0.132 | 1.54 |
| 8.00 | 0.511 | 5.95 |
| 15.00 | 0.587 | 6.85 |
| 28.00 | 0.693 | 8.07 |
| 43.00 | 0.795 | 9.26 |
| 58.00 | 0.894 | 10.42 |
| 79.00 | 1.028 | 11.99 |

FIGURE 1

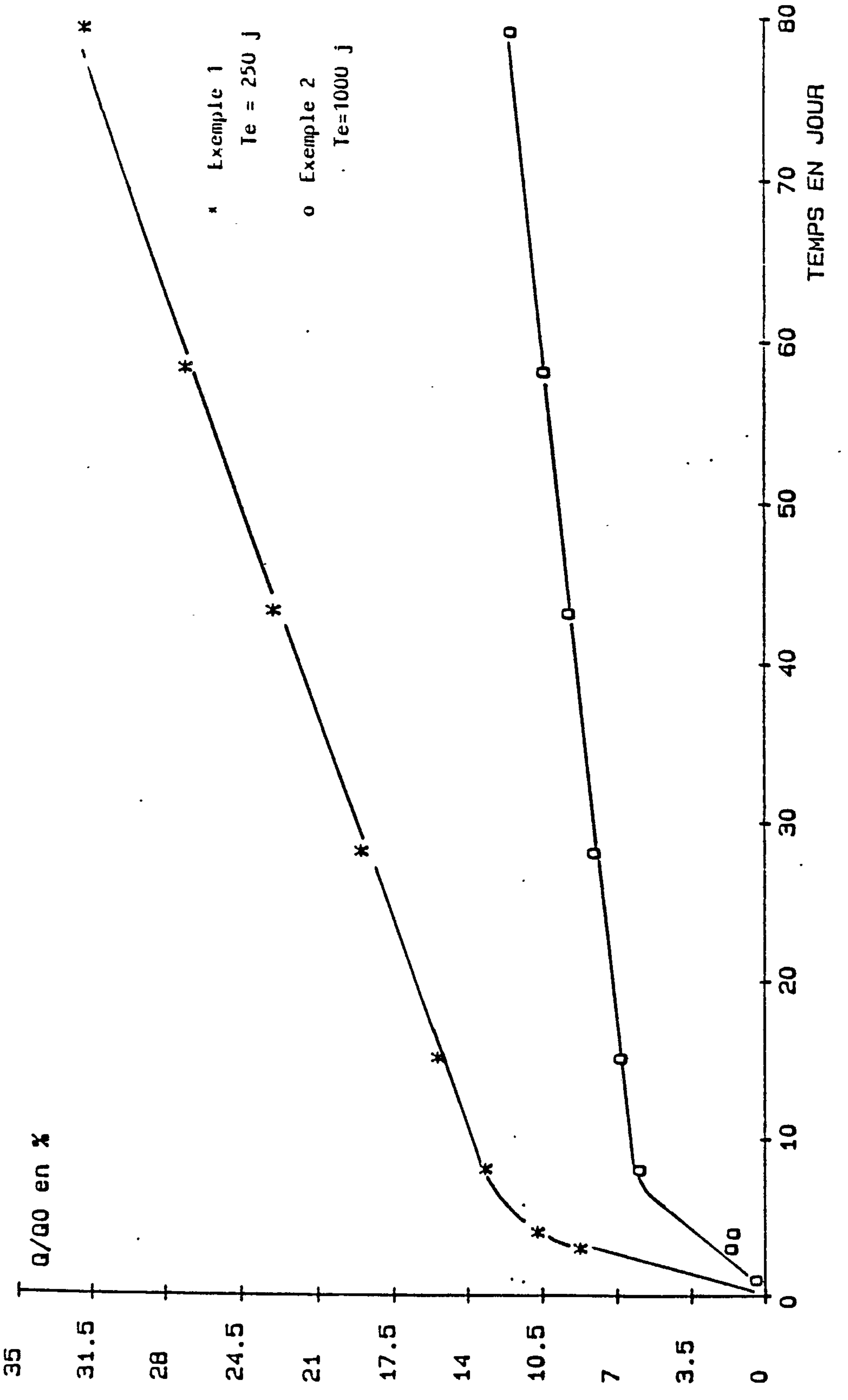


## Revendications

1 - Composition de silicone comportant :

A) Une huile diorganopolysiloxane, de viscosité inférieure à 300 000 mPa.s à 25° C,

B) Une charge minérale,

C) Un peroxyde organ

D) Au moins un composé organique et/ou minéral de l'iode sous forme solide ou liquide à température ambiante, soluble dans l'eau et non toxique.

2 - Composition selon la revendication 1, caractérisée en ce qu'elle contient, en poids, de 5 à 130, de préférence de 10 à 90 parties de composé de l'iode (D) pour 100 parties de l'huile (A).

3 - Composition selon la revendication 1 ou 2, caractérisé en ce que la charge minérale (B) est une charge siliceuse.

4 - Composition selon l'une quelconque des revendications précédentes, caractérisée en ce que le composé de l'iode (D) est un iodure ou iodate de formules générales :

$(M^{a+}) (I^{-})a$

et $(M^{a+}) (IO^{-}_{3})_{a}$

dans lesquelles a est un nombre entier supérieur ou égal à 1 et M est un cation choisi parmi un métal alcalin, un métal alcalino-terreux, un métal de transition et un ammonium quaternaire $(NY_4)^+$ dont les radicaux Y identiques ou différents représentent un radical alkyle linéaire ou ramifié en $C_1$-$C_{20}$ ou un atome d'hydrogène.

5 - Composition selon l'une quelconque des revendications précédentes, caractérisée en ce que le composé de l'iode (D) est choisi parmi

NA I, $Na IO_3$,

K I, $K IO_3$,

$Mg I_2$, $Mg I_2, 8H_2O$,

$Mg(IO_3)_2, 4H_2O$,

$NH_4 I$

$Fe I_2, 4H_2O$

$Mn I_2$

6 - Composition selon l'une quelconque des revendications 1 à 3, caractérisée en ce que le composé de l'iode (D) est l'iodobéhénate de calcium.

7 - Composition selon l'une quelconque des revendications 1 à 3, caractérisé en ce que le composé de l'iode (D) est la polyvinylpyrrolidone iodée.

8 - Composition selon l'une quelconque des revendications précédentes, caractérisée en ce qu'elle comporte :

- (A) 100 parties d'une huile (ou d'un mélange d'huiles) polysiloxane dont la viscosité à 25°C est inférieure à 300 000 mPa.s, de préférence comprise entre 800 et 250 000 mPa.s.
- (B) 5 à 90 parties d'une charge minérale, de préférence siliceuse, choisie parmi les silices de pyrogénation et les silices de précipitation.
- (C) 0,1 à 6 parties d'un peroxyde organique,
- (D) 5 à 130 parties, de préférence 10 à 90 parties d'un composé organique et/ou minéral de l'iode, soluble dans l'eau et non toxique.
- (E) 0 à 20 parties, de préférence 1 à 15 parties, d'un agent antistructure.

9 - Composition selon l'une quelconque des revendications précédentes caractérisée en ce que l'huile (A) est un polymère linéaire constitué essentiellement d'une succession de motifs de formule $R_2SiO$ et bloqué à chaque extrémité de sa chaîne par des motifs de formule $R_2R'SiO_{0,5}$, dans lesquelles les symboles R, identiques ou différents représentent des radicaux hydrocarbonés ayant de 1 à 8 atomes de carbone, substitués ou non par des atomes d'halogènes et/ou des groupes cyano, le symbole R' ayant la signification des symboles R ou représentant un radical hydroxyle, un radical alcoxy ayant de 1 à 4 atomes de carbone, β -méthoxyéthyle.

10 - Composition selon la revendication 9, dans laquelle les radicaus hydrocarbonés ayant de 1 à 8 atomes de carbone englobent :

- les radicaux alcoyles et halogénoalcoyles,
- les radicaux alcényles,
- les radicaus cycloalcoyles et halogénocycloalcoyles,
- les radicaux aryles et halogénoaryles,
- les radicaux cyanoalcoyles.

11 - Composition selon l'une quelconque des revendications précédentes caractérisée en ce qu'elle a une pénétration s'étalant de 80 à 400 avant l'incorporation du composé de l'iode.

12 - Composition selon l'une quelconque des revendications précédentes, caractérisée en ce qu'elle est pompable.

13 - Composition selon l'une quelconque des revendications précédentes durcie en un élastomère par chauffage.

14 - Procédé de traitement d'eaux, caractérisé en ce qu'on immerge une quantité adaptée de l'élastomère telle que définie à la revendication 13 en vue de libérer dans l'eau une quantité continue et contrôlée d'iode.

Office européen des brevets

# RAPPORT DE RECHERCHE EUROPEENNE

Numero de la demande

EP 89 42 0271

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | FR-A-2 611 733 (RHONE-POULENC)<br>* En entier * & EP-A-283 407; EP-A-285 525<br>--- | 1-14 | A 61 K 9/20<br>C 08 K 13/02<br>C 02 F 1/76 |
| P,X | FR-A-2 611 734 (RHONE-POULENC)<br>* En entier * & EP-A-283 408<br>--- | 1-14 | |
| P,X | FR-A-2 611 735 (RHONE-POULENC)<br>* En entier * & EP-A-284 521 *<br>----- | 1-14 | |
| | | | **DOMAINES TECHNIQUES RECHERCHES (Int. Cl.5)** |
| | | | A 61 K<br>C 08 K<br>C 02 F |

Le présent rapport a été établi pour toutes les revendications

| Lieu de la recherche | Date d'achèvement de la recherche | Examinateur |
|---|---|---|
| LA HAYE | 06-11-1989 | VAN AKOLEYEN H.T.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)